# EUROPÄISCHE PATENTSCHRIFT

(11) **EP 2 903 660 B1**
(45) Veröffentlichungstag und Bekanntmachung des Hinweises auf die Patenterteilung: **25.11.2020**
(21) Anmeldenummer: 13776989.9
(22) Anmeldetag: 02.10.2013
(51) Int. Cl.: A61M 5/00, A61M 5/31, B65D 81/20, B65D 81/24, B65D 81/26

(54) **APPLIKATIONSANORDNUNG MIT EINEM ARZNEISTOFFFLUID**
APPLICATION ASSEMBLY WITH A MEDICAL FLUID
AGENCEMENT D'APPLICATION AVEC UN LIQUIDE PHARMACEUTIQUE

(30) Priorität: 04.10.2012 EP 12187273; 04.10.2012 US 201261709657 P
(43) Veröffentlichungstag der Anmeldung: 12.08.2015
(73) Patentinhaber: Fresenius Kabi Austria GmbH, 8055 Graz (AT)
(72) Erfinder: KERSCHBAUMER, Andreas, 6300 Wörgl/Tirol (AT); GORGES, Roland, A-8010 Graz (AT); GRIGOLEIT, Patricia, 65193 Wiesbaden (DE); KRENN, Christian, A-8132 Pernegg (AT); SCHLÖGL, Johann, A-4073 Wilhering (AT)
(74) Vertreter: Fresenius Kabi Deutschland GmbH
(86) Internationale Anmeldenummer: PCT/EP2013/070570
(87) Internationale Veröffentlichungsnummer: WO 2014/053560

(56) Entgegenhaltungen:
- EP-A1- 1 818 069
- EP-A1- 2 371 406
- EP-A1- 2 495 002
- DE-C1- 10 036 832
- JP-A- 2008 067 989
- US-A1- 2006 275 336
- US-A1- 2012 143 144

## Beschreibung

Die vorliegende Erfindung betrifft das Gebiet der Arzneistoffapplikation.

Zur Applikation von Arzneistoffen werden üblicherweise Applikationsspritzen verwendet, welche entweder manuell oder unter Verwendung eines Pumpensystems betätigbar sind. Herkömmliche Applikationsspritzen umfassen einen Behälter, welcher mit einem Arzneistoff befüllbar ist, sowie einen Kolben, welcher in dem Behälter zur Applikation des Arzneistoffs verschiebbar gelagert ist.

Mit einer Applikationsspritze applizierbare Arzneistoffe können in der Gestalt einer Arzneistoffemulsion vorliegen, mit welcher ein Behälter einer Applikationsspritze befüllbar ist. Bei sauerstoffempfindlichen Arzneistoffen wie Propofol werden diese Arzneistoffemulsionen daher üblicherweise in Glasgebinden, wie etwa Glasflaschen oder Ampullen, gelagert. So kann der Arzneistoff bis kurz vor der Anwendung sauerstoffdicht gelagert und bei Bedarf in eine Applikationsspritze mit einem Behälter überführt und dann appliziert werden. Eine derartige Lagerung von Arzneistoffen ist jedoch aufwändig und somit teuer. Außerdem können durch das gesonderte Umfüllen Verunreinigungen des Arzneistoffs entstehen.

In der Druckschrift WO 94/25089 wurde daher vorgeschlagen, Applikationsspritzen mit Glasbehältern zu verwenden, um darin unmittelbar das gegenüber Sauerstoff empfindliche Propofol zu lagern. Applikationsspritzen mit Glasbehältern sind jedoch nicht bruchsicher. Dadurch ist das Einspannen derartiger Glasbehälter in Pumpenspritzsysteme problematisch.

In dem Dokument JP 2008 067989 A ist ein Aufbewahrungsbehälter für eine vorgefüllte Spritze beschrieben. Der Spritzenkörper ist aus Polypropylen oder einem zyklischen Polyolefin hergestellt. Die Spritze und eine Kolbenstange sind in dem Aufbewahrungsbehälter eingelegt. Der Aufbewahrungsbehälter ist durch eine abziehbare Folie verschlossen.

In der Patentanmeldung US 2012/0143144 A1 ist ein Autoinjektor beschrieben, welcher einen Spritzenkörper mit einem Wirkstoff und einen Sauerstoffabsorber umfasst. Ein für Sauerstoff impermeabler Behälter umschließt den Wirkstoff und das Sauerstoff absorbierende Material in dem Autoinjektor.

In dem Dokument US 2006/0275336 A1 ist ein Kit für ein osteoinduktives Mittel beschrieben. Zwei Spritzen sind in einem Behälter mit einem von Sauerstoff freien Gas eingelegt.

Es ist daher die Aufgabe der vorliegenden Erfindung, ein effizientes Konzept zur Lagerung von sauerstoffempfindlichen Arzneistoffen in Behältern von Applikationsspritzen zu schaffen.

Diese Aufgabe wird durch die Merkmale der unabhängigen Ansprüche gelöst. Vorteilhafte Weiterbildungsformen sind Gegenstand der abhängigen Ansprüche, der Beschreibung sowie der Zeichnungen.

Die vorliegende Erfindung basiert auf der Erkenntnis, dass die obige Aufgabe durch die Verwendung eines mit sauerstoffempfindlichem Arzneistoff vorbefüllten Kunststoffbehälters einer Applikationsspritze, welcher durch eine sauerstoffdichte Hülle sauerstoffdicht umhüllt ist, gelöst werden kann. Auf diese Weise wird ermöglicht, sauerstoffempfindliche Arzneistoffe auch über eine längere Zeitdauer in vorbefüllten und sauerstoffdurchlässigen Kunststoffbehältern von Applikationsspritzen zu lagern.

Die sauerstoffdichte Hülle kann beispielsweise als Umverpackung oder Blister die Applikationsspritze mit dem vorbefüllten Kunststoffbehälter aufnehmen und gegen das Eindringen von Sauerstoff schützen.

Gemäß einem Aspekt betrifft die Erfindung eine Applikationsanordnung mit einer Applikationsspritze mit einem Kunststoffbehälter, wobei der Kunststoffbehälter mit einem sauerstoffempfindlichen Arzneistofffluid gefüllt ist, und einer sauerstoffdichten Hülle, welche die Applikationsspritze sauerstoffdicht umhüllt, vorzugsweise wobei eine Innenseite des Kunststoffbehälters wenigstens abschnittsweise silikonisiert ist. Durch diese Silikonisierung kann insbesondere die Gleitfähigkeit eines Kolbens in dem Kunststoffbehälter erhöht und das Arzneistofffluid zusätzlich geschützt werden.

Gemäß einem weiteren Aspekt betrifft die Erfindung eine Applikationsanordnung, mit einer Applikationsspritze umfassend einen Kunststoffbehälter, wobei der Kunststoffbehälter mit einem sauerstoffempfindlichen Arzneistofffluid gefüllt ist; und einer sauerstoffdichten Hülle, welche die Applikationsspritze sauerstoffdicht umhüllt, wobei das sauerstoffempfindliche Arzneistofffluid eine Arzneistoffemulsion ist und Propofol umfasst und wobei der Kunststoffbehälter aus Kunststoff geformt ist, der wenigstens ein Cyclo-Olefin-Copolymer umfasst und wobei eine Innenseite des Kunststoffbehälters wenigstens abschnittsweise silikonisiert ist. Ein derartiger Kunststoffbehälter ist insbesondere gegen Propofol, das als Lösungsmittel wirkt, widerstandsfähig. Gleichzeitig ist das Propofol insbesondere vor dem in der Umgebung vorhandenen Sauerstoff wirksam geschützt.

Die sauerstoffdichte Hülle umschließt bevorzugt die Applikationsspritze mit dem befüllten Kunststoffbehälter, wodurch auch bei sauerstoffdurchlässigen Kunststoffen der in dem Kunststoffbehälter gelagerte Arzneistoff gegen Sauerstoff geschützt werden kann. Der durch die Hülle umschlossene Innenraum, in welchem die Applikationsspritze angeordnet ist, kann zusätzlich evakuiert sein, um die Sauerstoffdichte zu reduzieren. Der durch die Hülle umschlossene Innenraum ist jedoch auch mit einem Fluid, wie Gas oder Flüssigkeit, befüllt, das sauerstoffarm ist oder Sauerstoff bindet.

Die sauerstoffdichte Hülle besitzt eine Sauerstoffdurchlässigkeit von kleiner als oder gleich 10 cm³/m² d bar, bevorzugt von kleiner als oder gleich 5 cm³/m² d bar, besonders bevorzugt kleiner als oder gleich 2 cm³/m² d bar. Die Sauerstoffdurchlässigkeit oder Sauerstoffdichtheit richtet sich nach den Angaben in der Norm DIN 53380.

Ferner stellt die sauerstoffdichte Hülle auch eine Barriere für Wasserdampf bereits. Die sauerstoffdichte Hülle besitzt eine Wasserdampfdurchlässigkeit von kleiner als oder gleich 10 g/m² d, bevorzugt von kleiner als oder gleich 5 g/m² d, besonders bevorzugt kleiner als oder gleich 3 g/m² d. Die Wasserdampfdurchlässigkeit richtet sich nach den Angaben in der Norm DIN 53122.

Der Kunststoffbehälter kann mit dem Arzneistofffluid vollständig oder nur teilweise gefüllt sein.

Die Applikationsspritze kann alleine durch den gefüllten Kunststoffbehälter gebildet sein. Gemäß einer Ausführungsform kann die Applikationsspritze eine Kunststoffkolbenstange umfassen. Die Kunststoffkolbenstange kann ein Bestandteil der Applikationsanordnung sein. Gemäß einer Ausführungsform ist die Kunststoffkolbenstange jedoch kein Bestandteil der Applikationsanordnung.

Gemäß einer Ausführungsform ist das sauerstoffempfindliche Arzneistofffluid eine Arzneistoffemulsion.

Gemäß einer Ausführungsform umfasst das Arzneistofffluid einen Arzneistoff mit schlechter Wasserlöslichkeit, wobei zum Lösen von 1 g Arzneistoff mindestens 30 ml Wasser benötigt werden. Somit ist es durch die Verwendung der sauerstoffdichten Hülle möglich, eine ganze Gruppe von sauerstoffempfindlichen Arzneistoffen dauerhaft zu lagern.

Gemäß einer Ausführungsform ist oder umfasst das Arzneistofffluid Propofol, insbesondere eine Propofol-Emulsion. Propofol wird beschrieben durch den chemischen Namen 2,6-Diisopropylphenol (IUAPC),

Der Kunststoffbehälter ist aus Kunststoff geformt, welcher eines der folgenden Polymere umfasst: Cyclo-Olefin-Copolymer, Cyclo-Olefin-Polymer oder Crystal Clear Polymer. Ein derartiger Kunststoffbehälter ist gegen Lösungsmittel widerstandsfähig. Insbesondere kann ein derartiger Kunststoffbehälter zur Lagerung von Propofol, das als Lösungsmittel wirkt, verwendet werden.

Gemäß einer Ausführungsform umfasst die Applikationsanordnung oder Applikationsspritze ferner eine in dem Kunststoffbehälter verschiebbar lagerbare Kunststoffkolbenstange, und die Kunststoffkolbenstange ist insbesondere neben dem Kunststoffbehälter angeordnet und durch die sauerstoffdichte Hülle sauerstoffdicht umhüllt.

Gemäß einer Ausführungsform kann die Applikationsspritze im Wesentlichen nur den befüllten Kunststoffbehälter umfassen, welcher sauerstoffdicht umhüllt ist. Die Kunststoffkolbenstange kann dann separat bereitgestellt werden. Zur Verpackung der Kunststoffkolbenstange ist gemäß dieser Ausführungsform keine sauerstoffdichte Hülle notwendig.

Gemäß einer Ausführungsform ist die Kunststoffkolbenstange aus Kunststoff geformt, welcher eines der folgenden Polymere umfasst: Cyclo-Olefin-Copolymer, Cyclo-Olefin-Polymer oder Crystal Clear Polymer, oder sie ist aus Polypropylen geformt.

In einer weiteren Ausgestaltung der Erfindung ist bzw. sind der Kunststoffbehälter und/oder die Kunststoffkolbenstange in der sauerstoffdichten Hülle zumindest axial verschiebbar gelagert. Dadurch können der Kunststoffbehälter und/oder die Kunststoffkolbenstange bei einem seitlichen, beispielsweise axialen, Stoß eine Ausgleichsbewegung durchführen, zum Beispiel in Richtung der gegenüberliegenden Seite, insbesondere ohne dass sie selbst beschädigt werden und/oder ohne dass die Schale beschädigt wird. In einer Ausgestaltung der Erfindung ist bzw. sind der Kunststoffbehälter und/oder die Kunststoffkolbenstange und die Hülle so ausgeführt, dass die beiden Stirnseiten des Kunststoffbehälters und/oder die beiden Stirnseiten der Kunststoffkolbenstange jeweils in einem Abstand von etwa 0,5 cm bis etwa 2 cm von der Innenseite der Hülle entfernt positionierbar sind. Dadurch wird ausreichend Platz bereitgestellt, um eine axiale Ausgleichsbewegung zu ermöglichen.

In einer weiteren Ausführungsform der Erfindung sind der Kunststoffbehälter und die Kunststoffkolbenstange zumindest durch wenigstens einen flexiblen, vorzugsweise elastischen, Abstandshalter getrennt voneinander gelagert. Dadurch können Stöße in einer Richtung quer zur Längsachse des Kunststoffbehälters und/oder der Kunststoffkolbenstange abgefedert werden. Der wenigstens eine Abstandshalter besitzt in einer Ausführungsform eine Breite von etwa 0,3 cm bis etwa 1,5 cm.

Gemäß einer Ausführungsform umfasst die sauerstoffdichte Hülle eine Ethylen-Vinylalkohol-Copolymer-Barierre, insbesondere eine Ethylen-Vinylalkohol-Copolymer-Schicht. Durch das Vorsehen der Ethylen-Vinylalkohol-Copolymer-Barriere wird die Hülle sauerstoffdicht gemacht. Die Ethylen-Vinylalkohol-Copolymer-Barriere kann beispielsweise in der Gestalt einer Ethylen-Vinylalkohol-Copolymer-Schicht, mit welcher die Hülle versiegelt ist, ausgestaltet sein. Die Ethylen-Vinylalkohol-Copolymer-Barriere kann jedoch direkt in Kunststoff vorgesehen sein, aus dem die sauerstoffdichte Hülle hergestellt sein kann.

Gemäß einer Ausführungsform kann die sauerstoffdichte Hülle aus mehrschichtigem Kunststoff aufgebaut sein. Die Kunststoffschichten können beispielsweise eine Polyethylenterephthalat-Folie mit einer Ethylen-Vinylalkohol-Copolymer-Barriere oder Polyester mit einer Ethylen-Vinylalkohol-Copolymer-Barriere umfassen. Der mehrschichtige Kunststoff kann ferner eine peelfähigen Polyethylenfolie, insbesondere mit einer Ethylen-Vinylalkohol-Copolymer-Barriere sauerstoffdicht versiegelt, umfassen.

Gemäß einer Ausführungsform umfasst die sauerstoffdichte Hülle eine Schale, welche aus thermoplastischem Kunststoff geformt ist, insbesondere tiefgezogen ist. Die Schale kann beispielsweise tiefgezogen sein, um die Applikationsspritze aufzunehmen.

Gemäß einer Ausführungsform formt oder bildet die Schale ein Formteil für die formschlüssige Aufnahme der Applikationsspritze. Auf diese Weise wird die Applikationsspritze formschlüssig in der Schale gehalten. Das die Schale formende Formteil kann ferner eine formschlüssige Aufnahme für die Kolbenstange umfassen. Die jeweilige formschlüssige Aufnahme kann jeweils durch eine Einmuldung in einem Schalenboden realisiert werden. Die Schale kann ferner so geformt sein, dass eine Verrastung der Applikationsspritze darin möglich ist. So kann die als Formteil gebildete Schale beispielsweise seitlich Rastvorsprünge umfassen, welche elastisch sind und beim Einlegen des Kunststoffbehälters und/oder der Kunststoffkolbenstange zunächst verdrängt werden und danach in ihre ursprüngliche Position zurückkehren, wodurch die Applikationsspritze arretiert werden kann. In einer Ausgestaltung besitzt die Schale eine Wanddicke in einem Bereich von 50 µm bis 5000 µm, vorzugsweise von 10 µm bis 1000 µm. Die Messung erfolgt gemäß DIN 53370.

Gemäß einer Ausführungsform umfasst der thermoplastische Kunststoff eine amorphe Polyethylenterephthalat-Folie mit einer Ethylen-Vinylalkohol-Copolymer-Barierre oder biaxial orientiertes Polyester mit einer Ethylen-Vinylalkohol-Copolymer-Barierre. In einer Ausgestaltung besitzt der thermoplastische Kunststoff, welcher die Schale formt, ein Flächengewicht in einem Bereich von 50 g/m² bis 5000 g/m², vorzugsweise von 300 g/m² bis 1000 g/m². Die Messung erfolgt gemäß ISO 2286-2.

Gemäß einer Ausführungsform ist die Schale mit einer peelfähigen Folie oder Polyethylenfolie oder einer Polyethylenfolie mit einer Ethylen-Vinylalkohol-Copolymer-Barierre sauerstoffdicht versiegelt. In einer Ausgestaltung besitzt die peelfähige Folie eine Dicke in einem Bereich von 10 µm bis 2000 µm, vorzugsweise von 50 µm bis 300 µm. Die Messung erfolgt gemäß DIN 53370. In einer Ausgestaltung besitzt die peelfähige Folie ein Flächengewicht in einem Bereich von 10 g/m² bis 1000 g/m², vorzugsweise von 50 g/m² bis 200 g/m². Die Messung erfolgt gemäß ISO 2286-2.

Gemäß einer Ausführungsform umfasst die sauerstoffdichte Hülle eine weitere aus thermoplastischem Kunststoff geformte Schale, insbesondere eine aus thermoplastischem Kunststoff geformte Schale, wobei die weitere Schale eine Ethylen-Vinylalkohol-Copolymer-Barierre umfasst, und wobei die weitere Schale mit der Schale sauerstoffdicht verbunden ist, insbesondere verschweißt oder verklebt ist. Die weitere Schale kann beispielsweise ebenfalls ein Formteil für die formschlüssige Aufnahme der Applikationsspritze formen. Die Applikationsspritze wird somit einerseits durch eine ein Unterteil formende Schale und andererseits durch einen Deckel oder durch ein Oberteil, welches durch die weitere Schale geformt ist, gehalten. Die Schale und die weitere Schale sind beispielsweise entlang einer Verbindungsnaht sauerstoffdicht verbunden, was durch Verschweißen oder Verkleben realisiert werden kann. Die miteinander verbundenen Schalen formen somit eine stabile Hülle, welche den vorgefüllten Kunststoffbehälter sicher aufnehmen kann. Die weitere Schale kann ebenfalls aus Kunststoff ausgebaut sein und denselben Materialaufbau wie die Schale aufweisen.

Gemäß einer Ausführungsform ist der Kunststoffbehälter distal und proximal durch jeweils einen lösbaren Verschluss fluiddicht verschlossen. Die lösbaren Verschlüsse können beispielsweise als Stopfen oder Pfropfen oder Kolben oder als Schraubverschlüsse ausgebildet sein. Dadurch wird verhindert, dass das Arzneistofffluid ausläuft. Der distalseitige Verschluss des Kunststoffbehälters kann durch einen Kolben gebildet sein, in welchen eine Kunststoffkolbenstange einsetzbar oder einschraubbar ist.

Gemäß einer Ausführungsform kann die Applikationsanordnung einen Sauerstoffabsorber, der beispielsweise Eisenstaub umfassen kann, umfassen. Der Sauerstoffabsorber kann in die Schale der Hülle einlegbar sein oder mit der Schale verbunden sein.

Die Innenseite des Kunststoffbehälters und/oder die Außenseite des Kolbens und/oder die Außenseite des Verschlusses ist wenigstens abschnittsweise, vorzugsweise vollständig, silikonisiert, insbesondere mit Silikon beschichtet. Dadurch erhöht sich deren Gleitfähigkeit. Darüber hinaus wird dadurch der Arzneistoff zusätzlich geschützt.

Gemäß einem weiteren Aspekt betrifft die Erfindung ein Verfahren zum Herstellen der erfindungsgemäßen Applikationsanordnung. Das Verfahren umfasst die Schritte des Bereitstellens einer Applikationsspritze mit einem Kunststoffbehälter, des Befüllens des Kunststoffbehälters mit einem sauerstoffempfindlichen Arzneistofffluid, insbesondere mit Propofol, und des sauerstoffdichten Umhüllens der Applikationsspritze mit dem befüllten Kunststoffbehälter durch eine sauerstoffdichte Hülle.

Weitere Merkmale des Herstellungsverfahrens ergeben sich unmittelbar aus dem Aufbau der erfindungsgemäßen Applikationsanordnung und den insbesondere zur Herstellung der vorgenannten Kunststoffe notwendigen Herstellungsschritten.

Weitere Ausführungsformen werden Bezug nehmend auf die beiliegenden Zeichnungen näher erläutert. Es zeigen:
- Fig. 1: eine schematische Ansicht einer Applikationsanordnung;
- Fig. 2a, b: schematische Seitenansichten einer Applikationsanordnung und;
- Fig. 3: ein Ablaufdiagramm eines Herstellungsverfahrens zur Herstellung einer Applikationsanordnung.

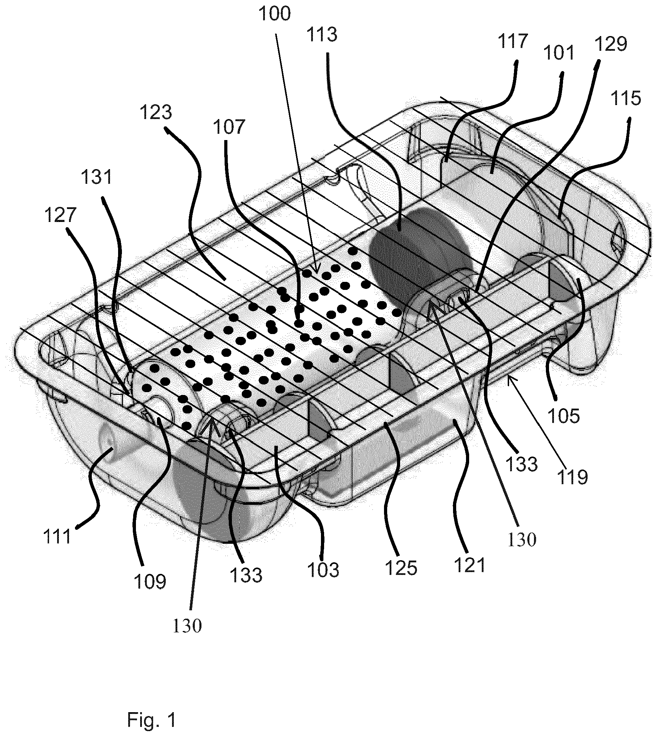

Fig. 1 zeigt schematisch eine Applikationsanordnung gemäß einer Ausführungsform. Die Applikationsanordnung umfasst eine Applikationsspritze 100 mit einem Kunststoffbehälter 101 und einer Kunststoffkolbenstange 103. Die Kolbenstange 103 umfasst an ihrem distalen Ende einen Einsatz 105, welcher in einen Kolben 113 einführbar ist. Die Kunststoffkolbenstange 103 ist hier beispielsweise außerhalb des Kunststoffbehälters 101 gelagert.

Der Kunststoffbehälter 101 ist, wie in Fig. 1 angedeutet, mit einem Arzneistofffluid 107, vorzugsweise mit Propofol, gefüllt. Der Kunststoffbehälter 101 umfasst proximalseitig eine Düse 109, welche mit einem Verschluss 111, beispielsweise mit einer Kunststoffkappe, verschlossen ist. Die Düse 109 weist einen, vorzugsweise männlichen, Luer-Anschluss oder Luer-Lock-Anschluss auf.

Der Kunststoffbehälter 101 ist distalseitig mit einem Kolben 113 abgeschlossen, in welchen der Einsatz 105 einführbar, beispielsweise eindrehbar ist. Auf diese Weise kann die Kunststoffkolbenstange 103 um den Kolben 113 vervollständigt werden. Der Kolben 113 dient somit gleichzeitig auch als ein distaler Verschluss des Kunststoffbehälters 101. Gemäß einer Ausführungsform kann die Kunststoffkolbenstange 103 statt des Einsatzes 105 bereits einen Kolben aufweisen. In diesem Fall ist der Kolbenbehälter 101 distalseitig mit einem weiteren Verschluss versehen.

Weiterhin distalseitig umfasst der Kunststoffbehälter 101 Seitenflügel 115, 117, welche neben einer manuellen Applikation auch eine Verwendung der Applikationsspritze in einem Pumpensystem ermöglichen.

Die Applikationsanordnung umfasst ferner eine sauerstoffdichte Hülle 119, welche beispielsweise als Blister oder als Umverpackung gebildet sein kann und die Applikationsspritze 100 vollständig und sauerstoffdicht umhüllt.

Die sauerstoffdichte Hülle 119 umfasst eine sauerstoffdichte Schale 121 sowie eine sauerstoffdichte Folie 123, welche die Schale 121 sauerstoffdicht entlang eines Randes 125 der Schale 121 versiegelt.

Hierzu kann die Schale 121 aus einer transparenten Tiefziehfolie auf Basis einer APET-Folie mit einer EVOH-Barriere (EVOH: Ethylen-Vinylalkohol) und einer peelfähigen Siegelschicht aus Polyethylen geformt sein. Die Schale 121 ist somit eine gute Sauerstoffbarriere und verfügt über eine hohe Steifigkeit sowie über ein gutes Tiefziehverhalten, was eine Ausgestaltung der Schale 121 als ein Formteil ermöglicht. Die Schale 121 kann zumindest eine der folgenden technischen Eigenschaften aufweisen:
Gesamtdicke 500 µm DIN 53370, Flächengewicht 646 g/m² ISO 2286-2,
Sauerstoffdurchlässigkeit < 1 cm³/m² d bar 23°C / 35% r.F. DIN 53380,
Wasserdampfdurchlässigkeit < 1 g/m² d 23°C / 85% r.F. DIN 53122.

Die Folie 123 kann beispielsweise eine Polyethylenfolie mit einer EVOH-Barriere sein und dadurch ebenfalls sauerstoffdicht sein. Die Folie 123 kann ferner ebenfalls auf Basis einer APET-Folie (APET: amorphes Polyethylenterephthalat) hergestellt sein, wodurch eine erhöhte Steifigkeit realisiert werden kann. Die Folie 123 ist beispielsweise entlang des Randes 125 mit der Schale 121 sauerstoffdicht durch beispielsweise Verschweißen oder Verkleben verbunden.

Die Schale 121 kann als Formteil zur formschlüssigen Aufnahme des Kunststoffbehälters 101 und des Kolbens 113 vorgesehen sein. Hierzu kann die Schale 121 eine Aufnahmemulde 127 für die Aufnahme des Kunststoffbehälters 101 und eine Aufnahmemulde 129 für die Aufnahme der Kunststoffkolbenstange 103 umfassen.

Zur Halterung des Kunststoffbehälters 101 kann die Aufnahmemulde 127 Klemmvorsprünge 131 umfassen. Die Aufnahmemulde 129 kann zur Halterung der Kunststoffkolbenstange 103 Klemmvorsprünge 133 umfassen. Die Klemmvorsprünge 131, 133 sind elastisch und somit beim Anlegen des Kunststoffbehälters 101 bzw. der Kunststoffkolbenstange 103 eindrückbar. Durch die Elastizität der Klemmvorsprünge 131, 133 üben diese eine beispielsweise radial gerichtete Kraft auf den Kunststoffbehälter 101 bzw. auf die Kunststoffkolbenstange 103 aus, wodurch diese Elemente fixiert werden können. Die Elemente 101 und/oder 103 können in einer Ausführungsform so fixiert sein, dass sie aus der Halterung nicht herausfallen können, aber gleichzeitig noch axial verschiebbar sind. Dadurch kann bzw. können der Kunststoffbehälter 101 und/oder die Kunststoffkolbenstange 103 zum Beispiel bei einem axialen Stoß eine axiale Ausgleichsbewegung durchführen, insbesondere ohne dass sie selbst beschädigt werden und/oder ohne dass die Schale 121 beschädigt wird.

Die Aufnahmemulde 127 für die Aufnahme des Kunststoffbehälters 101 und die Aufnahmemulde 129 für die Aufnahme der Kunststoffkolbenstange 103 sind durch wenigstens einen Abstandshalter 130 getrennt voneinander angeordnet. In der gezeigten Ausführungsform sind sie durch zwei Abstandshalter 130 getrennt voneinander angeordnet. An den Abstandshaltern 130 sind die vorstehend genannten Klemmvorsprünge 131, 133 angeordnet. Die Abstandshalter 130 sind flexibel, vorzugsweise elastisch. Dadurch können Stöße in einer Richtung quer zur Längsachse des Kunststoffbehälters 101 und/oder der Kunststoffkolbenstange 103 abgefedert werden. Die beiden Abstandshalter 130 werden jeweils durch eine Art Mauer- oder Wandabschnitt bereitgestellt.

Die Schale 121 und/oder die Folie 123 ist aus einem Kunststoffverbund aus biaxial orientiertem Polyester mit einer koextrudierten Siegelsperrschicht aus Polyethylen, EVOH, Polyethylen geformt. Ein derartiger Kunststoffverbund zeichnet sich durch eine gute Transparenz aus. Darüber hinaus sind ein breiter Siegelbereich sowie eine gute Sauerstoffbarriere realisierbar. Eine derart geformte Folie 123 kann insbesondere als Deckelfolie zur Versiegelung von tiefgezogenen Schalen, beispielsweise der Schale 121, eingesetzt werden. Die Folie 123 kann zumindest eine der folgenden technischen Eigenschaften aufweisen:
Gesamtdicke 100 µm DIN 53370, Flächengewicht 107,5 g/m² ISO 2286-2, Reißfestigkeit-Längs 40 - 60 N/mm² ISO 527-3, Reißfestigkeit-Quer 35 - 55 N/mm² ISO 527-3,
Reißdehnung-Längs 40 - 120 % ISO 527-3, Reißdehnung-Quer 40 - 120 % ISO 527-3,
Sauerstoffdurchlässigkeit 1 cm³/m² d bar 23°C / 35% r.F. DIN 53380, CO2 Durchlässigkeit < 4 cm³/m² d bar 23°C / 35% r.F. DIN 53380, N2 Durchlässigkeit < 1 cm³/m² d bar 23°C / 35% r.F. DIN 53380.

Der Kunststoffbehälter 101 ist gemäß einer Ausführungsform aus cyclischem Polymer geformt. Cyclische Polymere weisen eine hohe Reinheit sowie eine Resistenz auf, was eine längere Lagerung von Arzneistoffen gewährleistet. Cyclische Polymere sind jedoch sauerstoffdurchlässig bzw. sauerstoffpermeabel. Diese Sauerstoffpermeabilität ist nicht nur für sauerstoffempfindliche Produkte, wie Öl-in-Wasser-Emulsionen, sondern auch für sauerstoffempfindliche Arzneistoff, wie Propofol, nachteilig.

Zur Kompensation dieser Sauerstoffpermeabilität des Kunststoffbehälters 101 gemäß einer Ausführungsform ist die Hülle 119 vorgesehen, welche aus Kunststoffblister aufgebaut sein kann und eine Sperrschicht gegenüber Sauerstoff aufweisen kann.

Der Kunststoffbehälter 101 ist gemäß einer Ausführungsform aus COC (Cyclic Olefin Copolymer), COP (Cyclic Olefin Polymer) oder CCP (Crystal Clear Polymer) geformt. Diese erweisen sich als resistent gegenüber Propofol, das als Lösungsmittel wirkt. Der Kolben 113 und/oder der Verschluss 111 ist beispielsweise aus Gummi, insbesondere aus Bromobutyl, geformt. Die Kunststoffkolbenstange 103 kann beispielsweise aus Polypropylen geformt sein.

Der Kunststoffbehälter 101 ist aus einem transparenten Kunststoff aus der Gruppe von Cyclo-Olefin-Copolymeren oder Cyclo-Olefin-Polymeren geformt. Die Hülle 119 formt eine sauerstoffundurchlässige Verpackung, welche den mit dem Arzneistofffluid 107 befüllten Kunststoffbehälter 101 umschließt.

Die Innenseite des Kunststoffbehälters 101 ist silikonisiert bzw. mit Silikon beschichtet. Dadurch erhöht sich deren Gleitfähigkeit. Darüber hinaus wird der Arzneistoff dadurch zusätzlich geschützt.

Gemäß einer weiteren Ausführungsform ist Kolben 113 zumindest abschnittsweise silikonisiert. Gemäß einer Ausführungsform ist auch der Verschluss 11 zumindest abschnittsweise silikonisiert.

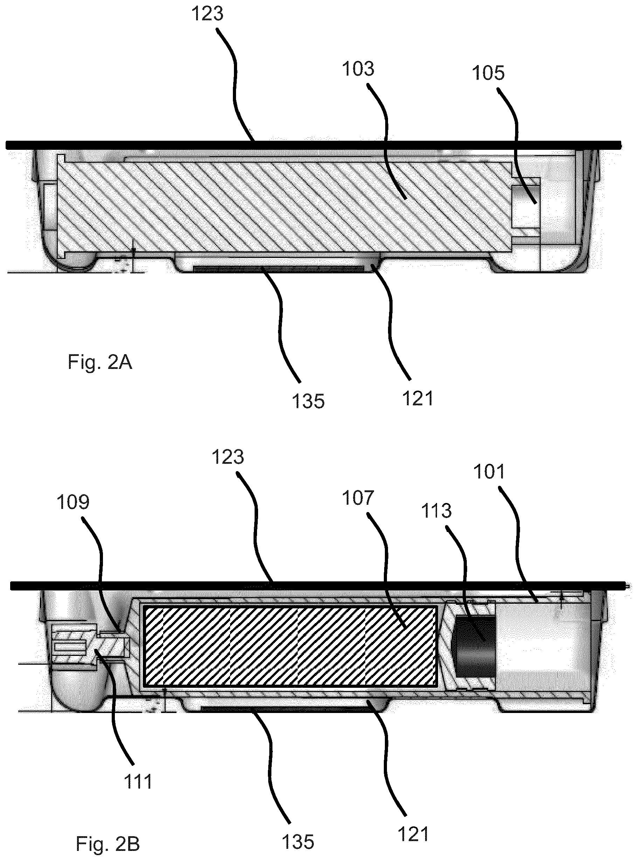

In den Fig. 2a und 2b sind unterschiedliche Seitenansichten der in Fig. 1 dargestellten Applikationsanordnung dargestellt. Gemäß einer Ausführungsform kann die Applikationsanordnung einen nicht fluiden Sauerstoffabsorber 135, der beispielsweise Eisenstaub umfassen kann, umfassen. Der Sauerstoffabsorber 135 ist beispielsweise am Boden der Schale 121 angeordnet.

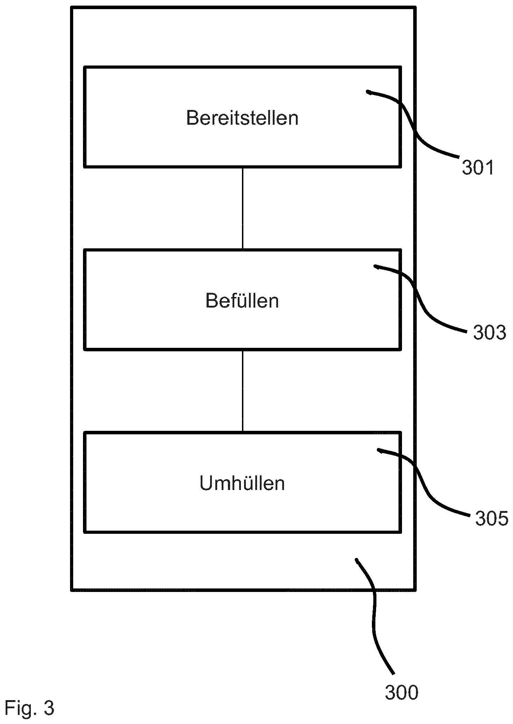

In Fig. 3 ist ein Diagramm eines Verfahrens zur Herstellung einer Applikationsanordnung dargestellt. Das Verfahren 300 umfasst den Schritt 301 des Bereitstellens einer Applikationsspritze mit einem Kunststoffbehälter, den Schritt 303 des Befüllens des Kunststoffbehälters mit einem sauerstoffempfindlichen Arzneistofffluid, beispielsweise mit Propofol oder mit einem Propofol-Fluid, insbesondere einer Propofol-Emulsion, und sauerstoffdichtes Umhüllen 305 der Applikationsspritze mit dem befüllten Kunststoffbehälter durch eine sauerstoffdichte Hülle. Das Verfahren umfasst ferner den Schritt des Anordnens des vorbefüllten Kunststoffbehälters 101 sowie der Kunststoffkolbenstange 103 in der tiefgezogenen Schale 121 sowie das sauerstoffdichte Verschließen der Schale 121 mit der Deckelfolie 123 durch beispielsweise Verschweißen oder Verkleben.

Ein Vorteil der Applikationsanordnung ist gemäß einer Ausführungsform deren einfache Anwendung ohne die Notwendigkeit einer Übertragung eines Arzneistoffs in weitere Gebinde. Darüber hinaus kann die Applikationsspritze direkt entnommen und angewendet werden, ohne dass weitere Montageschritte notwendig sind. Der Kunststoffbehälter 101 hat beispielsweise eine Kapazität von 50 ml, wodurch eine automatische Erkennung des Kunststoffbehälters durch herkömmliche Pumpensysteme möglich ist. Durch die Verwendung des Kunststoffbehälters wird eine gegenüber Glas erhöhte Bruchsicherheit erreicht. Darüber hinaus kann eine Abfallmenge reduziert werden bei einem gleichzeitigen Zeitgewinn durch eine Eliminierung von Applikationsvorbereitungsschritten, welche beispielsweise durch einen Transfer des Arzneistoffs bedingt sind.

### Bezugszeichenliste

- 100: Applikationsspritze
- 101: Kunststoffbehälter
- 103: Kunststoffkolbenstange
- 105: Einsatz
- 107: Arzneistofffluid
- 109: Düse
- 111: Verschluss
- 113: Kunststoffkolben
- 115: Seitenflügel
- 117: Seitenflügel
- 119: Sauerstoffdichte Hülle
- 121: Schale
- 123: Folie
- 125: Rand
- 127: Aufnahmemulde für die Applikationsspritze
- 129: Aufnahmemulde für die Kolbenstange
- 130: Abstandshalter oder Wandabschnitt zwischen der Aufnahmemulde für die Applikationsspritze und der Aufnahmemulde für die Kolbenstange
- 131: Klemmvorsprung
- 133: Klemmvorsprung
- 135: Sauerstoffabsorber

- 300: Verfahren
- 301: Schritt des Bereitstellens
- 303: Schritt des Befüllens
- 305: sauerstoffdichtes Umhüllen

## Patentansprüche

1. Applikationsanordnung, mit:
- einer Applikationsspritze (100) umfassend einen Kunststoffbehälter (101) aus einem transparenten Kunststoff aus der Gruppe von Cyclo-Olefin-Copolymeren oder Cyclo-Olefin-Polymeren, wobei der Kunststoffbehälter (101) mit einem sauerstoffempfindlichen Arzneistofffluid (107) gefüllt ist; und
- einer sauerstoffdichten Hülle (119), welche die Applikationsspritze (100) sauerstoffdicht umhüllt, wobei die sauerstoffdichte Hülle (119) eine sauerstoffdichte Schale (121) und eine sauerstoffdichte Folie (123) umfasst, **dadurch gekennzeichnet,**
**dass** die Schale (121) aus einer transparenten Tiefziehfolie auf Basis einer APET-Folie mit einer EVOH-Barriere und einer peelfähigen Siegelschicht aus Polyethylen geformt ist und/oder dass die Folie (123) aus einem transparenten Kunststoffverbund aus biaxial orientiertem Polyester mit einer koextrudierten Siegelsperrschicht aus Polyethylen, EVOH, Polyethylen geformt ist,
wobei ein durch die Hülle (119) umschlossener Innenraum mit einem Fluid befüllt ist, das sauerstoffarm ist oder Sauerstoff bindet; und
- einem Sauerstoffabsorber (135) in der Hülle (119),
wobei eine Innenseite des Kunststoffbehälters (101) wenigstens abschnittsweise silikonisiert ist.

2. Applikationsanordnung nach Anspruch 1, wobei das sauerstoffempfindliche Arzneistofffluid (107) eine Arzneistoffemulsion ist.

3. Applikationsanordnung nach Anspruch 1 oder 2, wobei das Arzneistofffluid (107) einen Arzneistoff mit einer Wasserlöslichkeit umfasst, bei dem zum Lösen von 1 g Arzneistoff mindestens 30 ml Wasser benötigt werden.

4. Applikationsanordnung nach einem der vorstehenden Ansprüche, wobei das Arzneistofffluid (107) Propofol umfasst.

5. Applikationsanordnung nach einem der vorstehenden Ansprüche, wobei der Kunststoffbehälter (101) aus Kunststoff geformt ist, der Crystal Clear Polymer umfasst.

6. Applikationsanordnung nach einem der vorstehenden Ansprüche, wobei die Applikationsspritze (100) eine in dem Kunststoffbehälter (101) verschiebbar lagerbare Kunststoffkolbenstange (103), insbesondere eine in dem Kunststoffbehälter (101) verschiebbar lagerbare und neben dem Kunststoffbehälter (101) angeordnete Kunststoffkolbenstange (103), umfasst, welche durch die sauerstoffdichte Hülle (119) sauerstoffdicht umhüllt ist.

7. Applikationsanordnung nach Anspruch 6, wobei die Kunststoffkolbenstange (103) aus Kunststoff geformt ist, der wenigstens eines der folgenden Polymere umfasst: Cyclo-Olefin-Copolymer, Cyclo-Olefin-Polymer oder Crystal Clear Polymer, oder aus Polypropylen geformt ist.

8. Applikationsanordnung nach einem der vorstehenden Ansprüche, wobei der Kunststoffbehälter (101) in der sauerstoffdichten Hülle (119) zumindest axial verschiebbar gelagert ist und/oder wobei die Kunststoffkolbenstange (103) in der sauerstoffdichten Hülle (119) zumindest axial verschiebbar gelagert ist.

9. Applikationsanordnung nach einem der vorstehenden Ansprüche, wobei der Kunststoffbehälter (101) und die Kunststoffkolbenstange (103) zumindest durch wenigstens einen flexiblen Abstandshalter (130) getrennt voneinander gelagert sind.

10. Applikationsanordnung nach einem der vorstehenden Ansprüche, wobei die Schale (121) tiefgezogen ist.

11. Applikationsanordnung nach Anspruch 10, wobei die Schale (121) ein Formteil für die formschlüssige Aufnahme der Applikationsspritze (100) bildet.

12. Applikationsanordnung nach einem der Ansprüche 10 bis 11, wobei die sauerstoffdichte Hülle (119) eine weitere aus thermoplastischem Kunststoff geformte Schale, insbesondere eine aus thermoplastischem Kunststoff geformte Schale umfasst, wobei die weitere Schale eine Ethylen-Vinylalkohol-Copolymer-Barierre umfasst, und wobei die weitere Schale mit der Schale (121) sauerstoffdicht verbunden ist, insbesondere verschweißt oder verklebt ist.

13. Verfahren zum Herstellen der Applikationsanordnung gemäß einem der Ansprüche 1 bis 12, mit:
Bereitstellen (301) einer Applikationsspritze mit einem Kunststoffbehälter (101) aus einem transparenten Kunststoff aus der Gruppe von Cyclo-Olefin-Copolymeren oder Cyclo-Olefin-Polymeren;
Silikonisieren wenigstens eines Abschnitts einer Innenseite des Kunststoffbehälters (101);
Befüllen (303) des Kunststoffbehälters mit einem sauerstoffempfindlichen Arzneistofffluid, insbesondere mit Propofol; und
sauerstoffdichtes Umhüllen (305) der Applikationsspritze mit dem befüllten Kunststoffbehälter durch eine sauerstoffdichte Hülle (119), wobei die sauerstoffdichte Hülle (119) eine sauerstoffdichte Schale (121) und eine sauerstoffdichte Folie (123) umfasst, **dadurch gekennzeichnet,**
**dass** die Schale (121) aus einer transparenten Tiefziehfolie auf Basis einer APET-Folie mit einer EVOH-Barriere und einer peelfähigen Siegelschicht aus Polyethylen geformt ist und/oder dass die Folie (123) aus einem transparenten Kunststoffverbund aus biaxial orientiertem Polyester mit einer koextrudierten Siegelsperrschicht aus Polyethylen, EVOH, Polyethylen geformt ist,
wobei ein durch die Hülle (119) umschlossener Innenraum mit einem Fluid befüllt wird, das sauerstoffarm ist oder Sauerstoff bindet, und ein Sauerstoffabsorber (135) in die Hülle (119) eingelegt wird.

## Claims

1. An application assembly comprising:
- an applicator syringe (100) comprising a plastic receptacle (101) made of a transparent plastic from the group of cyclic olefin copolymers or cyclic olefin polymers, wherein the plastic receptacle (101) is filled with an oxygen-sensitive active pharmaceutical ingredient fluid (107); and
- an oxygen-tight envelope (119) which envelopes the applicator syringe (100) in an oxygen-tight manner, wherein the oxygen-tight envelope (119) comprises an oxygen-tight shell (121) and an oxygen-tight film (123), **characterized in that**
the shell (121) is formed from a transparent deep-drawn film based on APET film with an EVOH barrier and a peelable sealing layer made of polyethylene and/or **in that** the film (123) is formed from a transparent plastic composite of biaxially oriented polyester comprising a co-extruded sealing block layer made of polyethylene, EVOH, polyethylene,
wherein an interior enclosed by the envelope (119) is filled with a fluid which is low in oxygen or binds oxygen; and
- an oxygen absorber (135) in the envelope (119),
wherein an inside of the plastic receptacle (101) is siliconized at least in sections.

2. The application assembly according to claim 1, wherein the oxygen-sensitive active pharmaceutical ingredient fluid (107) is an active pharmaceutical ingredient emulsion.

3. The application assembly according to claim 1 or 2, wherein the active pharmaceutical ingredient fluid (107) comprises an active pharmaceutical ingredient having a water solubility in which at least 30 ml of water are required to dissolve 1 g of active pharmaceutical ingredient.

4. The application assembly according to any of the preceding claims, wherein the active pharmaceutical ingredient fluid (107) comprises propofol.

5. The application assembly according to any of the preceding claims, wherein the plastic receptacle (101) is formed from plastic comprising Crystal Clear Polymer.

6. The application assembly according to any of the preceding claims, wherein the applicator syringe (100) comprises a plastic plunger rod (103) storable in a shiftable manner in the plastic receptacle (101), in particular a plastic plunger rod (103) which is storable in a shiftable manner in the plastic receptacle (101) and arranged beside the plastic receptacle (101) and which is enveloped in an oxygen-tight manner by means of the oxygen-tight envelope (119).

7. The application assembly according to claim 6, wherein the plastic plunger rod (103) is formed from plastic comprising at least one of the following polymers: cyclic olefin copolymer, cyclic olefin polymer or Crystal Clear Polymer, or is formed from polypropylene.

8. The application assembly according to any of the preceding claims, wherein the plastic receptacle (101) is stored in an at least axially shiftable manner in the oxygen-tight envelope (119) and/or wherein the plastic plunger rod (103) is stored in an at least axially shiftable manner in the oxygen-tight envelope (119).

9. The application assembly according to any of the preceding claims, wherein the plastic receptacle (101) and the plastic plunger rod (103) are stored at least separately by means of at least one flexible spacer (130).

10. The application assembly according to any of the preceding claims, wherein the shell (121) is deep-drawn.

11. The application assembly according to claim 10, wherein the shell (121) forms a molding for the formfitting accommodation of the applicator syringe (100).

12. The application assembly according to any of claims 10 to 11, wherein the oxygen-tight envelope (119) comprises a further shell formed from thermoplastic, in particular a shell formed from thermoplastic, wherein the further shell comprises an ethylene-vinyl alcohol copolymer barrier, and wherein the further shell is joined, in particular welded or adhesively bonded, to the shell (121) in an oxygen-tight manner.

13. A method for producing the application assembly according to any one of claims 1 to 12, comprising:
Providing (301) an applicator syringe comprising a plastic receptacle (101) made of a transparent plastic from the group of cyclic olefin copolymers or cyclic olefin polymers;
Siliconizing at least one section of an inside of the plastic receptacle (101);
Filling (303) the plastic receptacle with an oxygen-sensitive active pharmaceutical ingredient fluid, in particular with propofol; and
Enveloping (305), in an oxygen-tight manner, the applicator syringe with the filled plastic receptacle by means of an oxygen-tight envelope (119), wherein the oxygen-tight envelope (119) comprises an oxygen-tight shell (121) and an oxygen-tight film (123), **characterized in that**
the shell (121) is formed from a transparent deep-drawn film based on APET film with an EVOH barrier and a peelable sealing layer made of polyethylene and/or **in that** the film (123) is formed from a transparent plastic composite of biaxially oriented polyester comprising a co-extruded sealing block layer made of polyethylene, EVOH, polyethylene,
wherein an interior enclosed by the envelope (119) is filled with a fluid which is low in oxygen or binds oxygen and an oxygen absorber (135) is laid into the envelope (119).

## Revendications

1. Dispositif d'application, comprenant :
- une seringue d'application (100) comprenant un réservoir en matière plastique (101) fait de matière plastique transparente du groupe de copolymères d'oléfine cyclique ou polymères d'oléfine cyclique, dans lequel le réservoir en matière plastique (101) est rempli d'un fluide d'ingrédient pharmaceutique actif (107) sensible à l'oxygène ; et
- une enveloppe (119) étanche à l'oxygène entourant la seringue d'application (100) de manière étanche à l'oxygène, dans laquelle l'enveloppe (119) étanche à l'oxygène comprend un boîtier (121) étanche à l'oxygène et une pellicule (123) étanche à l'oxygène, **caractérisé en ce que**
le boîtier (121) est formé à partir d'une pellicule transparente emboutie basée sur une pellicule APET avec une barrière EVOH et une couche de scellage pelable en polyéthylène et/ou **en ce que** la pellicule (123) est formée d'un composite de matière plastique transparente de polyester orienté biaxialement comprenant une couche barrière de scellage coextrudée en polyéthylène, EVOH, polyéthylène,
dans lequel un espace intérieur enfermé par l'enveloppe (119) est rempli avec un fluide qui est pauvre en oxygène ou fixe l'oxygène ; et
- un absorbeur d'oxygène (135) dans l'enveloppe (119),
dans lequel un côté intérieur du réservoir en matière plastique (101) est siliconé au moins en sections.

2. Dispositif d'application selon la revendication 1, dans lequel le fluide d'ingrédient pharmaceutique actif (107) sensible à l'oxygène est une émulsion d'ingrédient pharmaceutique actif.

3. Dispositif d'application selon la revendication 1 ou 2, dans lequel le fluide d'ingrédient pharmaceutique actif (107) comprend un ingrédient pharmaceutique actif ayant une solubilité dans l'eau dans lequel au moins 30 ml d'eau sont nécessaires pour dissoudre 1 g d'ingrédient pharmaceutique actif.

4. Dispositif d'application selon l'une quelconque des revendications précédentes, dans lequel le fluide d'ingrédient pharmaceutique actif (107) comprend du propofol.

5. Dispositif d'application selon l'une quelconque des revendications précédentes, dans lequel le réservoir en matière plastique (101) est formé à partir de plastique comprenant un polymère Crystal Clear.

6. Dispositif d'application selon l'une quelconque des revendications précédentes, dans lequel la seringue d'application (100) comprend une tige du piston en matière plastique (103) entreposable de manière déplaçable dans le réservoir en matière plastique (101), en particulier une tige du piston en matière plastique (103) entreposable de manière déplaçable dans le réservoir en matière plastique (101) et disposée à côté du réservoir en matière plastique (101), laquelle est enveloppée de manière étanche à l'oxygène au moyen de l'enveloppe (119) étanche à l'oxygène.

7. Dispositif d'application selon la revendication 6, dans lequel la tige du piston en matière plastique (103) est formée à partir de matière plastique comprenant au moins l'un des polymères suivants : copolymère d'oléfine cyclique, polymère d'oléfine cyclique ou polymère Crystal Clear, ou est formée à partir de polypropylène.

8. Dispositif d'application selon l'une quelconque des revendications précédentes, dans lequel le réservoir en matière plastique (101) est entreposé de manière au moins axialement déplaçable dans l'enveloppe (119) étanche à l'oxygène et/ou dans lequel la tige du piston en matière plastique (103) est entreposée de manière au moins axialement déplaçable dans l'enveloppe (119) étanche à l'oxygène.

9. Dispositif d'application selon l'une quelconque des revendications précédentes, dans lequel le réservoir en matière plastique (101) et la tige du piston en matière plastique (103) sont entreposés au moins séparément au moyen d'au moins un espaceur flexible (130).

10. Dispositif d'application selon l'une quelconque des revendications précédentes, dans lequel le boîtier (121) est embouti.

11. Dispositif d'application selon la revendication 10, dans lequel le boîtier (121) forme un moulage pour le logement selon une liaison par formes complémentaires de la seringue d'application (100).

12. Dispositif d'application selon l'une quelconque des revendications 10 à 11, dans lequel l'enveloppe (119) étanche à l'oxygène comprend un boîtier supplémentaire formé à partir de thermoplastique, en particulier un boîtier formé à partir de thermoplastique, dans lequel le boîtier supplémentaire comprend une barrière de copolymère d'éthylène-alcool vinylique, et dans lequel le boîtier supplémentaire est relié, en particulier soudé ou collé de manière adhésive, au boîtier (121) de manière étanche à l'oxygène.

13. Méthode de production du dispositif d'application selon l'une quelconque des revendications 1 à 12, comprenant :
L'approvisionnement (301) d'une seringue d'application comprenant un réservoir en matière plastique (101) fait d'une matière plastique transparente du groupe de copolymères d'oléfine cyclique ou polymères d'oléfine cyclique ;
Le siliconage d'au moins une section d'un côté intérieur du réservoir en matière plastique (101) ;
Le remplissage (303) du réservoir en matière plastique avec un fluide d'ingrédient pharmaceutique actif sensible à l'oxygène, en particulier avec du propofol ; et
L'enveloppement (305), de manière étanche à l'oxygène, de la seringue d'application avec le réservoir en matière plastique rempli au moyen d'une enveloppe (119) étanche à l'oxygène, dans laquelle l'enveloppe (119) étanche à l'oxygène comprend un boîtier (121) étanche à l'oxygène et une pellicule (123) étanche à l'oxygène, **caractérisée en ce que**
le boîtier (121) est formé à partir d'une pellicule transparente emboutie basée sur une pellicule APET avec une barrière EVOH et une couche de scellage pelable en polyéthylène et/ou **en ce que** la pellicule (123) est formée d'un composite de matière plastique transparente de polyester orienté biaxialement comprenant une couche barrière de scellage coextrudée en polyéthylène, EVOH, polyéthylène,
dans laquelle un espace intérieur enfermé par l'enveloppe (119) est rempli avec un fluide qui est pauvre en oxygène ou fixe l'oxygène, et un absorbeur d'oxygène (135) est placé dans l'enveloppe (119).
